# EUROPEAN PATENT APPLICATION

(11) **EP 4 417 126 A1**
(43) Date of publication of application: **21.08.2024**
(21) Application number: 23157031.8
(22) Date of filing: 16.02.2023
(51) Int. Cl.: A61B 5/259, A61B 5/291

(54) **ELECTRODE CARRIER AND METHOD OF PERFORMING ELECTROENCEPHALOGRAPHY (EEG) MEASUREMENTS**

(71) Applicant: Prolira B.V., 3584 CH Utrecht (NL)
(72) Inventor: WILLEMS, Annemarie Christine, 3584 CH UTRECHT (NL); VAN MERKERK, Rutger Olof, 3584 CH UTRECHT (NL)
(74) Representative: IPecunia

(57) **Abstract**

The present invention relates to an electrode carrier for electroencephalography (EEG) measurements, the carrier comprising:
- a flexible substrate for placement on a person's scalp,
- a plurality of electrodes for electroencephalography measurements,

characterized in that the plurality of electrodes comprises:
- a first electrode, for placement on a region of the person's scalp that is typically substantially free of hair, the first electrode comprising a non-adhesive conductive medium for contacting the person's scalp, and
- a second electrode, different from the first electrode, for placement on a region of the person's scalp that is typically substantially covered with hair, the second electrode comprising an adhesive conductive medium for contacting the person's scalp.

## Description

### Field of the invention

The present invention relates to an electrode carrier for electroencephalography (EEG) measurements. The present invention further relates to a method of performing electroencephalography (EEG) measurements for determining a parameter which is indicative for whether a person has acute encephalopathy and/or delirium or not, or is at risk of becoming encephalopathic and/or delirious or not.

### State of the art

Electroencephalography (EEG) measurements can be used beneficially for determining a brain state of a person. In recent developments, it was found that EEG can be used to determine whether a person has normal brain activity or has acute encephalopathy and/or a delirium or whether the person is at risk of becoming encephalopathic and/or delirious. Such an acute change in brain state is, for example, very common with elderly persons, as a disadvantageous effect of underlying medical conditions like lung infections or drug intoxication.

In EP 3 046 466 B1, it is disclosed that EEG signals relevant for the above described changing brain states can be recorded from the person's brain by means of at least two electrodes on different locations on the person's scalp. A first electrode is placed on a frontal region of the person's head, for example on the forehead, and a second electrode is placed on a parietal region of the person's head, for example on the crown. From the EEG signals, a deviation signal can be analysed that is indicative of the encephalopathic and/or delirious state.

EP 3 558 095 B1 discloses an electrode carrier for use in EEG measurements, which comprises a flexible substrate, to be bent to follow the contours of the person's scalp. The carrier comprises EEG electrodes, arranged on the substrate. One electrode is intended for placement at the person's forehead and another electrode is intended for placement at the person's crown. The carrier comprises adhesive pads, surrounding the electrodes, for attaching the electrodes to the person's scalp.

EP 4 084 671 A1 discloses a processing method for detecting and classifying a segment of a signal that is obtained from EEG measurements as a target signal segment or as a non-target signal segment. The method comprises a voting process to determine whether classification of a first detected segment of the signal as a target signal segment or classification of a second detected segment of the signal as a non-target signal segment is correct.

Although this known electrode carrier was found to be very accurate and reliable, it might bring discomfort for persons. During positioning or removal of the carrier, after the measurements are performed, the adhesive pads could possibly pull out hairs of the person's scalp, which may cause pain and trauma, as well as resistance to next, repetitive measurements.

### Object of the invention

It is therefore an object of the invention to provide an electrode carrier for EEG measurements that reduces the risks of having traumas when positioned on, and removed from, the person's scalp and/or that provides improvement measurements results, or at least to provide an alternative electrode carrier.

### Detailed description

The present invention provides an electrode carrier for electroencephalography (EEG) measurements, the carrier comprising:
- a flexible substrate for placement on a person's scalp,
- a plurality electrodes for electroencephalography measurements, wherein the electrodes are arranged on the substrate,
characterized in that, the plurality of electrodes comprises:
- a first electrode, for placement on a region of the person's scalp that is typically substantially free of hair, the first electrode comprising a non-adhesive conductive medium for contacting the person's scalp, and
- a second electrode, different from the first electrode, for placement on a region of the person's scalp that is typically substantially covered with hair, the second electrode comprising an adhesive conductive medium for contacting the person's scalp.

The present electrode carrier differs from existing EEG electrode carriers, in that it comprises two different types of electrodes, which are each selected on the basis of their intended position on the person's scalp. In both types of electrodes, a conductive medium is provided to improve the electrically conductive contact between the skin of the scalp and the electrodes, to facilitate the transmission of the EEG signals into the electrodes.

The first electrode is intended for placement on a region of the person's scalp that is typically substantially free of hair, for example on the forehead, or at least a region where the person's hair can be moved away easily to expose the skin. The presence of bare skin without hair may allow for the use of external adhesive members for attaching the electrode to the skin. During removal of these external adhesive members, no hairs will be pulled, providing for substantially atraumatic removal of the carrier.

The second electrode is intended for placement on a region of the person's scalp that is typically substantially covered with hair, for example on the crown. The use of external adhesive members would give a painful experience for the person upon removal of the electrode, since a large number of hairs, surrounding the electrode, would be pulled out of the person's scalp by the external adhesive members. The present invention thus provides that an adhesive conductive medium is used for attaching the electrode to the persons scalp. The adhesive conductive medium may be applied on a conductor of the second electrode only and no separate adhesive members need to be present around the electrode.

The second electrode is preferably placed on the skin directly without any hair in between, which means that the adhesive conductive medium will not come into substantial contact with hair. During removal of the carrier, the adhesive conductive medium is therefore not likely to pull out any hairs, thereby reducing the risk of having painful and traumatic removal of the carrier.

Furthermore, the adhesive tack of the present adhesive conductive medium may be selected relatively weak, which may imply that, even if the adhesive conductive medium were to be adhered to hair, it may detach from the hair, instead of pulling out the hair.

The use of different types of electrodes, especially different types of conductive medium, in the same electrode carrier is not a straightforward improvement. It is normal practice in the field of EEG to provide all electrodes at the best possible similarity, in order to achieve the highest signal quality. Hence, also the cited prior art documents mention a single type of electrodes to be used.

The present inventors have surprisingly found that the present carrier, with different types of conductive medium in the first electrode and the second electrode, still enables accurate measuring and reliable results. This is not a straightforward improvement for the person skilled in the art. Hence, the skilled person would not depart from the use of electrode carriers with only a single type of electrode, for example the same electrode on the forehead and on the crown of the person's scalp. The skilled person would expect this to give reduced signal quality.

The carrier comprises at least one of each of the types of electrodes, but may comprise more electrodes. Additional electrodes of both types may for example be provided as a reference, ground or further measurement electrode. However, the present invention may also invention further types of electrodes to be used, different from the first electrode and the second electrode.

The carrier may further comprise a connector, electrically connected to the electrodes, for connecting the electrode carrier to external amplification, storage and processing means, so that the EEG signals can be transferred from the electrodes towards the other means. To establish this connection, the carrier may further comprise conductor tracks, extending between the connector and the electrodes.

In an embodiment, the first electrode may be a frontal electrode, for placement on the frontal region of the person's scalp and the second electrode may be a parietal electrode, for placement on the parietal region of the person's scalp, for example on the crown.

The frontal electrode is intended for placement on the frontal region of the person's scalp, for example on the forehead. Typically, foreheads are substantially free of hair, or at least the person's hair can be moved away easily to expose the forehead skin. The presence of bare skin at the person's forehead may allow for the use of external adhesive members for attaching the electrode to the forehead. During removal of these external adhesive members, no hairs will be pulled, providing for substantially atraumatic removal of the carrier.

The parietal electrode is intended for placement on the parietal region of the person's scalp, for example on the crown. Crowns, on the other hand, are typically substantially covered with hair. The use of external adhesive members would give a painful experience for the person upon removal of the parietal electrode, since a large number of hairs, surrounding the electrode, would be pulled out of the person's scalp by the external adhesive members.

In the present carrier, the frontal electrode may be arranged on a front end of the substrate, seen with respect to the person's scalp in an applied state during EEG measurements, and the parietal electrode may be arranged on a rear end of the substrate, opposite to the front end.

In an embodiment, at least one of the electrodes, for example the frontal electrode further comprises transmission improvement means for improving the transmission of EEG signals from the person's brain, i.e. underneath the skin, towards the conductor of the electrode. Furthermore, the transmission improvement means may be beneficial in mitigating dermal effects occurring on the person's skin, such as sweating of the person, which would otherwise have a negative influence on the quality of the EEG measurements.

In a further embodiment, the transmission improvement means comprise tines for shallow penetration in the person's scalp, for example the stratum corneum of the person's skin. The tines may be provided in a large number, arranged adjacent to each other in a gridlike manner, protruding from a common, central body. The tines may be made of a plastic material, for example made of Nylon, to obtain sufficient strength for shallow penetration of the person's skin.

The tines may be arranged behind the non-adhesive conductive medium and in front of the conductor of the electrode, seen from the side of the carrier that is to be arranged on the person's skin, so that the tines will come to protrude through the conductive medium upon placement. As such, part of the conductive medium may be taken along with the tines, into the skin, to further facilitate the transmission of the EEG signals towards the electrodes.

In an embodiment, the non-adhesive conductive medium of the first electrode comprises a liquid, for example a liquid hydrogel. The non-adhesive conductive medium is thereby substantially free of significant adhesive properties. The liquid may be held in front of the conductor of that first electrode to facilitate transmitting the EEG signals towards the conductor of the electrode. The liquid, especially liquid hydrogel, may be selected to be optimal for transmitting the EEG signals towards the conductors of the electrodes, since it does not need to have any adhesive properties.

In a further embodiment, the liquid has a viscosity in the range between 1 cPs and 350 cPs, for example about 350 cPs. It was found by the inventors that a liquid with viscosity in this range may have a favourable contribution to the overall electric conductivity of the skin electrode, whilst having minimal adhesive properties to avoid sticking to the person's skin. Hence, adhesive properties of the first electrodes are provided by other materials, not being provided at the actual first electrode itself.

In an embodiment, at least one of the electrodes, for example the first electrode comprises a flexible matrix. The flexible matric is configured to deform when pressed against the person's skin. The flexible matrix may be made of an absorbing material, for example embodied as a sponge for holding the conductive medium. The absorbing flexible matrix is configured to hold the non-adhesive conductive medium at the conductor of the electrode. Hence, the non-adhesive conductive medium, for example the liquid, may be viscous and could thus flow from the conductors of the electrodes in the absence of any absorbing material.

In a further embodiment of the carrier, the transmission improvement means, for example the tines, can at some point protrude through the flexible matrix. The tines may be arranged behind the non-adhesive conductive medium and the flexible matrix and in front of the conductor of the electrode, seen from the side of the carrier that is to be arranged on the person's skin, so that the tines will come to protrude through the non-adhesive conductive medium and the flexible matrix upon placement of the carrier on the skin. As such, part of the conductive medium may be taken along with the tines, out of the flexible matrix and into the stratum corneum, to further facilitate the transmission of the EEG signals towards the electrodes.

The flexible matrix may further serve the purpose of pushing the tines out of the person's stratum corneum upon relaxation of the flexible matrix, back to its original shape. This may be beneficial, since the flexible matrix is able to gently pull the tines out and away from the person's skin.

In an embodiment, the carrier further comprises an adhesive pad for the first electrode, at least partially surrounding the first electrode, for securing the placement of the first electrode on the person's scalp. The adhesive pad is preferably made of an electrically insulating material, to prevent transmission of EEG signals through it. The first electrode can be free of adhesive parts, so as not to adhere to the person's skin. Instead, the present adhesive pad is able to provide for such adhesion, to hold the electrode against the persons skin.

The adhesive pad at least partially surrounds the first electrode, so that the adhesive forces from the adhesive pad can be provided at multiple different parts over at least part of the perimeter of the electrode. As such, the first electrode may be pressed against the person's skin from multiple points around its perimeter, to provide for an even pressing of the electrode on the skin.

The use of an adhesive pad is very suitable for the first electrode, since the foreheads of persons are generally free of hairs, so the adhesive pads will not have any disadvantageous effect on the person comfort, i.e. avoiding pulling hairs out of the person's skin.

In a further embodiment, the adhesive pad is attached to the substrate and fully surrounds the first electrode. The adhesive pad may thereby comprise a central aperture in which the electrode can be provided. As such, the electrode will be pressed against the person's skin along its entire perimeter, to provide for a further improved even pressing of the electrode on the skin.

In an embodiment, the adhesive pad comprises an adhesive foam pad. Such an adhesive foam pad may be beneficial, as it is generally deformable to accurately follow the contours of the persons forehead, when gently pressed against it, in order to maximize the adhesive properties of the adhesive foam pad

In an embodiment, the adhesive pad may have an adhesive tack in the range between 20 and 140 grams/mm, preferably between 50 and 110 grams/mm, for example 80 grams/mm to cello, measured according to the PSTC- 5 test method of the Pressure Sensitive Tape Council, wherein the adherence to cello is included as a reference material used for testing according to this standard method.

It was found that an adhesive tack in the range of this embodiment offers a desirable balance between sufficient adhesion, i.e. for reliably pressing the electrode against the forehead and for reliably holding the carrier, whilst minimizing the risk of having painful and traumatic removal of the carrier, i.e. when the adhesive pad is pulled-off the forehead.

In an embodiment, the adhesive conductive medium comprises a substantially solid medium, for example a solid hydrogel. The solid medium may be held in front of the conductor of the second electrode to facilitate transmitting the EEG signals towards the electrode. The solid medium, especially solid hydrogel, may be selected to be optimal for transmitting the EEG signals.

Furthermore, the solid medium may be selected to further have optimal adhesive properties, to ensure that the second electrode can be adhered to the person's skin, in the absence of further adhesive pads. As such, it can be prevented that separate adhesive members need to be in contact with hair of the person, during use.

In an embodiment, the solid medium has a viscosity in the range between 600 cPs and 3000 cPs, for example about 1000 cPs. It was found by the inventors that a solid medium with viscosity in this range may have a favourable contribution to the overall electric conductivity of the skin electrode.

In an embodiment, the solid medium has an adhesive tack larger than 8.5 grams/mm to stainless steel, measured according to the PSTC- 5 test method of the Pressure Sensitive Tape Council, wherein the adherence to stainless steel is included as a reference material.

It was found that an adhesive tack in the range of this embodiment offers a desirable balance between sufficient adhesion, i.e. for reliably pressing the electrode against the person's skin, i.e. crown, and for reliably holding the carrier in place, whilst minimizing any discomfort for the person during removal of the carrier, i.e. when the adhesive pad is pulled-off. Hence, the adhesive tack of the solid medium is selected to be sufficiently low, so that, even if the adhesive conductive medium were to be adhered to hair, it may detach from the hair, instead of pulling out the hair.

In an embodiment, the second electrode may have an aspect ratio larger than the aspect ratio of the first electrode. The aspect ratio may be defined as the ratio of the length of the electrode, divided by its width. This difference in aspect ratio provides that the second electrode has a more elongate shape, compared to the shape of the first electrode.

The large aspect ratio of the second electrode may provide the beneficial effect that the elongate second electrode can overlay a parting made in the person's hair, to minimize disturbances in the EEG measurements from hair underneath the electrode and to minimize discomfort of the adhesive conductive medium pulling out hairs when the carrier is removed from the person's scalp.

The aspect ratio of the second electrode may be in the range of 4 to 10, for example about 7, whereas the aspect ratio of the first electrode may be in the range of 1 to 4, for example about 2.

In a further embodiment, the second electrode is aligned parallel to a vertical direction, i.e. being substantially elongate in the vertical direction. It was found by the inventors that vertical placement of the second electrode, for example at a vertical parting in the person's hair, yielded especially usability aspects, i.e. better than, for example, horizontal placement.

In an embodiment, adhesive anchors are provided on the substrate for attaching the substrate to a side part of the person's scalp, the anchors preferably comprising a substantially solid medium, for example a solid hydrogel, which may be the same as the solid medium used to form the adhesive conductive medium in the second electrode. These anchors may be provided in addition to the adhesive pads that are included in the electrodes and serve the purpose of improving the overall adhesive tack of the carrier. This may provide that the carrier is not only supported from the person's head at the electrodes, but also in between them.

The anchors may prevent the need of full circumvention around the person's head, for example with a belt or cap, which is a common application method for attaching EEG electrodes. The option to enable montage to one side part of the person's scalp, instead of circumvention is advantageous for usability, in case of restricted access to the head due to, for example, restricted mobility of bedridden persons.

The use of anchors may be particularly beneficial where the carrier is provided with a connector in between the electrodes. The connector itself may have a certain weight that is to be supported, but also any cables extending towards the amplification, storage and processing means may be suspended from the connector. All this additional weight can be supported by the anchors attached to the substrate.

The present invention further provides a method of performing electroencephalography (EEG) measurements for determining a parameter which is indicative for whether a person has acute encephalopathy and/or a delirium or not, or is at risk of becoming encephalopathic and/or delirious or not, the method comprising the steps of:
- providing an electrode carrier as disclosed herein,
- placing the second electrode on a region of the person's scalp that is typically substantially free of hair, for example on the crown,
- mechanically adhering and electrically contacting the person's scalp with the second electrode, via the adhesive conductive medium,
- placing the first electrode on a region of the person's scalp that is typically substantially covered with hair, for example on the forehead,
- electrically contacting the person's scalp with the first electrode, via the non-adhesive conductive medium, and
- recording signals representative of brain activity from the first and second electrodes.

The present method may comprise one or more of the features and/or benefits disclosed herein in relation to the electrode carrier according to the present invention, especially as recited in the appended claims.

The present method of performing EEG measurements differs from existing EEG methods, in that it relies on a different type of electrode carrier, having two different types of electrodes for placement on both typically hairy and typically hairless parts of the patient's skin, and since the electrode carrier is adhered to the person's skin in a different manner.

The electrode carrier comprises two different types of electrodes, which are each selected on the basis of their intended position on the person's scalp. In both types of electrodes, a conductive medium is provided to improve the electrically conductive contact between the skin of the scalp and the electrodes, to facilitate the transmission of the EEG signals into the electrodes.

For placement of the electrode carrier on the person's head, the first electrode is placed on a region of the person's scalp that is typically substantially free of hair, for example on the forehead, or at least the person's hair can be moved away easily to expose the skin. The presence of bare skin without hair may allow for the use of external adhesive members for attaching the electrode to the skin. During removal of these external adhesive members, no hairs will be pulled, reducing the risk of having painful and traumatic removal of the carrier.

The second electrode is placed on a region of the person's scalp that is typically substantially covered with hair, for example on the crown thereof. The use of external adhesive members would give a painful or traumatic experience for the person, since a large number of hairs, surrounding the electrode, would be pulled out of the person's scalp. Also placing eventual external adhesive members surrounding the electrode on the hair, might cause the electrode to be lifted from the scalp due to the lifting effect of most hair types. The present invention thus provides that an adhesive conductive medium is used for attaching the second electrode to the persons scalp. The adhesive conductive medium is applied on a conductor of the electrode only and no separate adhesive member needs to be present around the electrode.

The second electrode comprises an adhesive conductive medium, so that the electrode itself is adhered to the person's skin, instead of adherence through additional adhesive pads. Furthermore, the adhesive tack of the present adhesive conductive medium may be selected weaker compared to that of the adhesive pads known from the prior art, which may imply that, even if the adhesive conductive medium were to be adhered to hair, it may detach from the hair, instead of pulling out the hair.

Both types of electrodes are thus placed on the person's skin, whereas the order of placement is of lesser importance. However, it can be favourable to first place the second electrode, so that it can be placed right after the person's hair is bent away, i.e. to form a parting, and after the skin is exposed.

As a final step of this method, after the electrode carrier is placed on the person's scalp, EEG signals are recorded by means of the electrodes. The EEG data signals are thereby recorded with the electrodes from at least two locations on the person's scalp. From the EEG data signals, a deviation signal can be analysed that is indicative of the encephalopathic and/or delirious state.

In an embodiment of the method, the first electrode may be a frontal electrode, for placement on the frontal region of the person's scalp and the second electrode may be a parietal electrode, for placement on the parietal region of the person's scalp, for example on the crown.

The frontal electrode is intended for placement on the frontal region of the person's scalp, for example on the forehead. Typically, foreheads are substantially free of hair, or at least the person's hair can be moved away easily to expose the forehead skin. The presence of bare skin at the person's forehead may allow for the use of external adhesive members for attaching the electrode to the forehead. During removal of these external adhesive members, no hairs will be pulled, providing for substantially atraumatic removal of the carrier.

The parietal electrode is intended for placement on the parietal region of the person's scalp, for example on the crown. Crowns, on the other hand, are typically substantially covered with hair. The use of external adhesive members would give a painful experience for the person upon removal of the parietal electrode, since a large number of hairs, surrounding the electrode, would be pulled out of the person's scalp by the external adhesive members.

In an embodiment, the method further comprises the step of securing the first electrode on the person's scalp with the adhesive pad. The conductive medium at the first electrode is non-adhesive, so the electrode itself is not adhered to the person's skin, in contrast to the adhesive conductive medium at the second electrode, used to adhere the second electrode to the person's scalp.

The present adhesive pad is used to secure the electrode carrier to the scalp at the first electrode as well. The use of such an adhesive pad is very suitable for the first electrode, since the foreheads of persons are generally free of hairs, so the adhesive pads will not have any disadvantageous effect on the person comfort, i.e. avoiding pulling hairs out of the person's skin.

In an embodiment, the method further comprises, prior to the step of placing of the second electrode, the step of forming of a parting in the person's hair at the person's crown. The parting may be formed to expose the person's skin from underneath the hair. The parting may preferably be formed in the vertical direction and the second electrode may be placed to be substantially elongate in the vertical direction, which was found to yield especially beneficial usability aspects.

The parting in the hair may be formed by means of a hair parting device, composed of two opposed patches that together define a central aperture, for example an elongate rectangular aperture, in between them. A first one of the patches may be used to bend a first, for example left, half of the hairs in a first direction. A second one of the patches may be slit over the first patch in a second direction, opposite to the first direction, to bend a second half of the hairs in the opposite direction. Accordingly, the parting is formed between both halves of bent-away hair.

### Brief description of drawings

Further characteristics of the invention will be explained below, with reference to embodiments, which are displayed in the appended drawings, in which:
Figure 1 schematically depicts an embodiment of the electrode carrier according to the present invention,

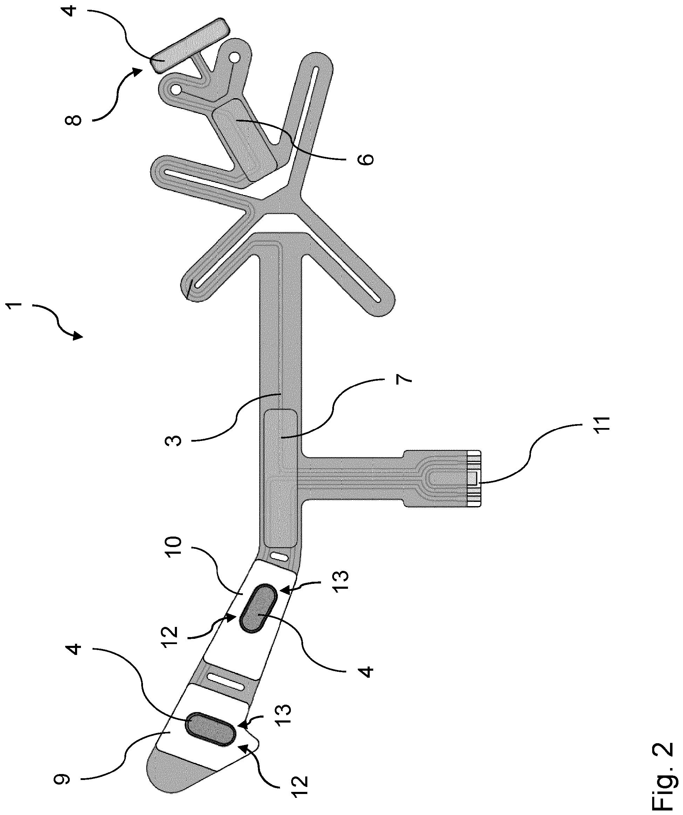

Figure 2 schematically depicts a cut-open view on the electrode carrier in figure 1, and

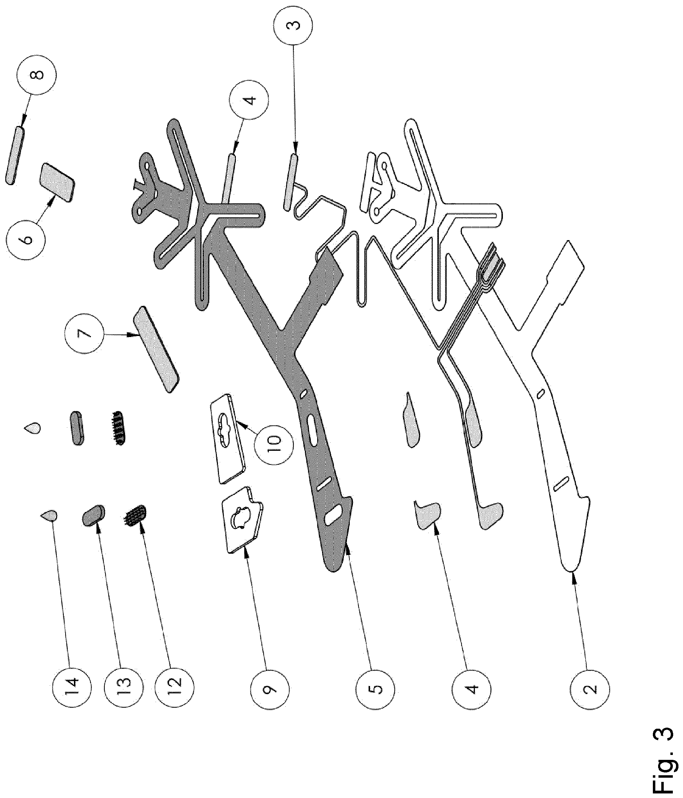

Figure 3 schematically depicts an exploded-view representation of the electrode carrier in figure 1.

Throughout the figures, the same reference numerals are used to refer to corresponding components or to components that have a corresponding function.

### Detailed description of embodiments

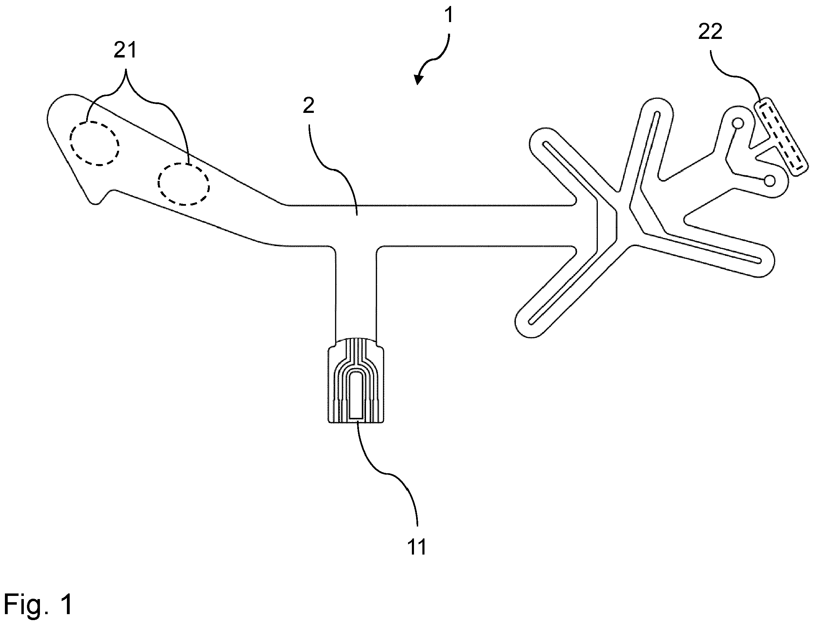

Figure 1 schematically depicts an embodiment of the electrode carrier for electroencephalography (EEG) measurements according to the present invention, to which is referred with reference numeral 1. The carrier 1 comprises a flexible substrate 2 for placement on a person's scalp. The carrier 1 comprises a plurality of electrodes 21, 22 arranged on the substrate 2, each comprising a conductor 4 for EEG measurements.

The carrier 1 comprises two first electrodes 21, i.e. frontal electrodes, arranged on a front end of the substrate 2, for placement on the frontal region of the person's scalp. One of the first electrodes 21 may act as a reference electrode.

The carrier further comprises a second electrode 22, i.e. a parietal electrode, arranged on a rear end of the substrate 2 and different from the frontal electrode 21, for placement on the crown of the person's scalp. In both types of electrodes 21, 22, a conductive medium is provided to improve the electrically conductive contact between the skin of the scalp and the conductors 4, to facilitate the transmission of the EEG signals into the conductors 4.

The carrier 1 further comprises a connector 11, electrically connected to the electrodes 20, 21, for connecting the electrode carrier 1 to external amplification, storage and processing means. This enables transfer of the EEG signals from the electrodes 21, 22 towards the other means. To establish this connection, the carrier 1 is provided with conductor tracks 3, extending between the connector 11 and the electrodes 21, 22.

The frontal electrodes 21 comprise a non-adhesive conductive medium 14, embodied as a liquid, which is held in front of the conductors 4 of the frontal electrodes 21 to facilitate transmitting the electromagnetic EEG signals towards the electrode 4. The liquid 14 has a viscosity of about 350 cPs.

The frontal electrodes 21 comprise tines 12 for securing the placement of the pads 21 on the person's scalp. The tines 12 are configured to interact with the skin of the person's forehead, which is beneficial in transmitting the EEG signals towards the conductors 4 and to mitigate electrical dermal effects occurring on the person's skin. The tines 12 allow for shallow penetration in the person's scalp, i.e. the stratum corneum of the skin. The tines 12 are made of Nylon and provided in a large number, arranged adjacent to each other in a gridlike manner, protruding from a common, central body.

The frontal electrodes 21 each comprise a flexible matrix of an absorbing material, embodied as a sponge 13, for holding the conductive medium 14. The sponge 13 is configured to hold the non-adhesive conductive medium 14 at the electrode 21. The tines 12 will thereby come to protrude through the non-adhesive conductive medium 14 and the sponge 13 upon placement of the carrier 1 on the skin. As such, part of the conductive medium 14 may be taken along with the tines 12, out of the sponge 13 and into the skin, to further facilitate the transmission of the EEG signals towards the electrodes 4.

The carrier 1 further comprises an adhesive pad 9, 10 for each of the frontal electrodes 21, which are attached to the substrate 2 and which fully surround the respective electrode 21. The adhesive pads 9, 10 are configured to secure the placement of the electrodes 21 on the person's scalp. The electrodes 21 are free of adhesive parts by themselves, so as not to adhere to the person's skin. Instead, the present adhesive pads 9, 10 are able to provide for such adhesion, to hold the electrode 21 against the persons head. The adhesive pads 9, 10 have an adhesive tack of about 80 grams/mm to cello, measured according to the PSTC- 5 test method of the Pressure Sensitive Tape Council.

The adhesive pads 9, 10 each comprise a central aperture in which the conductor 4 is provided. As such, the electrodes 21, 22 can be pressed against the person's skin along their entire perimeter, to provide for an improved and even pressing of the electrode 21, 22 against the skin. The adhesive pads are provided as adhesive foam pads 9, 10, being generally deformable to accurately follow the contours of the persons forehead, when gently pressed against it, in order to maximize the adhesive properties of the adhesive foam pads 9, 10.

The parietal electrode 22 comprises an adhesive conductive medium, embodied as a substantially solid hydrogel 8. The solid hydrogel 8 is held in front of the conductor 4 of the parietal electrode 22 to facilitate transmitting the electromagnetic EEG signals. The solid hydrogel 8 has an adhesive tack larger than 8.5 grams/mm to stainless steel, measured according to the PSTC- 5 test method of the Pressure Sensitive Tape Council, to reliably hold the carrier 1 on the person's head.

The parietal electrode 22 has an aspect ratio larger than the aspect ratio of the frontal electrode 21, which aspect ratio is defined as the ratio of the length of the electrode 22, divided by its width. The parietal electrode 22 thus has a more elongate shape, compared to the shape of the frontal electrodes 21. The parietal electrode 22 and the substrate 2 are relatively flexible, so that they can accurately follow the contours of the person's head.

The substrate 2 further comprises adhesive anchors 6, 7 for attaching the substrate 2 to a side part of the person's scalp. The anchors 6, 7 are provided as a solid hydrogel, which is the same as the solid hydrogel used to form the adhesive conductive medium 8 in the parietal electrode 22.

## Claims

1. Electrode carrier for electroencephalography (EEG) measurements, the carrier comprising:
- a flexible substrate for placement on a person's scalp,
- a plurality of electrodes for electroencephalography measurements, wherein the electrodes are arranged on the substrate,
**characterized in that,**
the plurality of electrodes comprises:
- a first electrode, for placement a region of the person's scalp that is typically substantially free of hair, the first electrode comprising a non-adhesive conductive medium for contacting the person's scalp, and
- a second electrode, different from the first electrode, for placement on a region of the person's scalp that is typically substantially covered with hair, the second electrode comprising an adhesive conductive medium for contacting the person's scalp.

2. Electrode carrier according to claim 1, wherein the first electrode is a frontal electrode, for placement on the frontal region of the person's scalp, and
wherein the second electrode is a parietal electrode, for placement on the parietal region of the person's scalp, for example on the crown.

3. Electrode carrier according to claim 1 or 2, wherein at least one of the electrodes, for example the frontal electrode further comprises transmission improvement means for improving the transmission of EEG signals from the person's brain, i.e. underneath the skin, towards the electrode.

4. Electrode carrier according to claim 3, wherein the transmission improvement means comprise tines for shallow penetration in the person's scalp, for example the stratum corneum of the person's skin.

5. Electrode carrier according to any of the preceding claims, wherein the non-adhesive conductive medium of the first electrode comprises a liquid, for example a liquid hydrogel.

6. Electrode carrier according to any of the preceding claims, wherein at least one of the electrodes, for example the first electrode comprises a flexible matrix, such as a sponge, for holding the non-adhesive conductive medium.

7. Electrode carrier according to claim 3 and claim 6, wherein the transmission improvement means, for example the tines, protrude through the flexible matrix.

8. Electrode carrier according to any of the preceding claims, further comprising an adhesive pad for the first electrode, at least partially surrounding the first electrode, for securing the placement of the first electrode on the person's scalp.

9. Electrode carrier according to claim 8, wherein the adhesive pad is attached to the substrate and fully surrounds the respective electrode.

10. Electrode carrier according to claim 8 or 9, wherein the adhesive pad comprises an adhesive foam pad.

11. Electrode carrier according to any of the preceding claims, wherein the adhesive conductive medium comprises a substantially solid medium, for example a solid hydrogel.

12. Electrode carrier according to claim 11, wherein the solid medium has an adhesive tack larger than 8.5 grams/mm to stainless steel, measured according to the PSTC-5 test method.

13. Electrode carrier according to any of the preceding claims, wherein the substrate comprises adhesive anchors for attaching the substrate to a side part of the person's scalp, the adhesive anchors preferably comprising a substantially solid medium, for example a solid hydrogel.

14. Method of performing electroencephalography (EEG) measurements, i.e. for determining a parameter which is indicative for whether a person has acute encephalopathy and/or a delirium or not, or is at risk of becoming encephalopathic and/or delirious or not, the method comprising the steps of:
- providing an electrode carrier according to any of the preceding claims,
- placing the second electrode on a region of the person's scalp that is typically substantially free of hair,
- mechanically adhering and electrically contacting the person's scalp with the second electrode, via the adhesive conductive medium,
- placing the first electrode on a region of the person's scalp that is typically substantially covered with hair,
- electrically contacting the person's scalp with the first electrode, via the non-adhesive conductive medium, and
- recording signals representative of brain activity from the first and second electrodes.

15. Method according to claim 14, further comprising the step of securing the first electrode on the person's scalp with the adhesive pad.
